# DEMANDE DE BREVET EUROPEEN

(11) **EP 3 211 096 A1**
(43) Date de publication de la demande: **30.08.2017**
(21) Numéro de dépôt: 16305226.9
(22) Date de dépôt: 26.02.2016
(51) Int. Cl.: C12Q 1/68

(54) **COMBINAISON DE MIARNS PERMETTANT LA PREDICTION DE L'EVOLUTION CLINIQUE DU CANCER DU RECTUM**

(71) Demandeur: Prestizia, 34830 Clapiers (FR); Institut Regional du Cancer de Montpellier (ICM), 34298 Montpellier Cedex 5 (FR)
(72) Inventeur: VETTER-GENOUD, Guillaume, 34920 Le Crès (FR); PRIGNEAU, Odile, 77170 Brie-Comte-Robert (FR); TOSOLINI, Laurent, 47440 Casseneuil (FR); VIEIRA, Gilles, 92340 Bourg la Reine (FR); HO-PUN-CHEUNG, Alexandre, 34380 Saint Martin de Londres (FR); MOLLEVI, Caroline, 34380 Mas de Londres (FR); CRAPEZ, Evelyne, 34980 Combaillaux (FR); ASSENAT, Eric, 34430 Saint Jean de Védas (FR)
(74) Mandataire: Cabinet Becker et Associés

(57) **Abrégé**

La présente demande concerne des compositions et méthodes pour analyser et caractériser des patients atteints d'un cancer du rectum basées notamment sur la détection de miARNs circulants. Elle est utilisable chez tout patient, et tout particulièrement chez les patients ayant un cancer de stade II ou III.

SEQUENCE LISTING

<110>  PRESTIZIA
       ICM

<120>  COMBINAISON DE MIARNS PERMETTANT LA PREDICTION DE L'EVOLUTION
       CLINIQUE DU CANCER COLORECTAL

<130>  B2193

<160>  26

<170>  PatentIn version 3.3

<210>  1
<211>  22
<212>  RNA
<213>  Artificial

<220>
<223>  miARN

<400>  1
uggagagaaa ggcaguuccu ga                                              22


<210>  2
<211>  21
<212>  RNA
<213>  Artificial

<220>
<223>  miARN

<400>  2
aacauagagg aaauuccacg u                                               21


<210>  3
<211>  23
<212>  RNA
<213>  Artificial

<220>
<223>  miARN

<400>  3
aaaagugcuu acagugcagg uag                                             23


<210>  4
<211>  22
<212>  RNA
<213>  Artificial

<220>
<223>  miARN

<400>  4
cauugcacuu gucucggucu ga                                              22


<210>  5

<211>  22
<212>  RNA
<213>  Artificial

<220>
<223>  miARN

<400>  5
uguaaacauc cucgacugga ag                                              22


<210>  6
<211>  22
<212>  RNA
<213>  Artificial

<220>
<223>  miARN

<400>  6
uagcagcacg uaaauauugg cg                                              22


<210>  7
<211>  22
<212>  RNA
<213>  Artificial

<220>
<223>  miARN

<400>  7
ugagguagua gguugugugg uu                                              22


<210>  8
<211>  23
<212>  RNA
<213>  Artificial

<220>
<223>  miARN

<400>  8
ugugcaaauc uaugcaaaac uga                                             23


<210>  9
<211>  23
<212>  RNA
<213>  Artificial

<220>
<223>  miARN

<400>  9
caaagugcuu acagugcagg uag                                             23


<210>  10
<211>  21
<212>  RNA
<213>  Artificial

<220>
<223>  miARN

<400>  10
ugaguuggcc aucugaguga g                                                21


<210>  11
<211>  22
<212>  RNA
<213>  Artificial

<220>
<223>  miARN

<400>  11
aaaccguuac cauuacugag uu                                               22


<210>  12
<211>  23
<212>  RNA
<213>  Artificial

<220>
<223>  miARN

<400>  12
ugugcaaauc caugcaaaac uga                                              23


<210>  13
<211>  22
<212>  RNA
<213>  Artificial

<220>
<223>  miARN

<400>  13
ugucaguuug ucaaauaccc ca                                               22


<210>  14
<211>  23
<212>  RNA
<213>  Artificial

<220>
<223>  miARN

<400>  14
ugugcaaauc uaugcaaaac uga                                              23


<210>  15
<211>  21
<212>  RNA
<213>  Artificial

<220>
<223>  miARN

<400>  15
uagcagcaca gaaauauugg c                                                21

<210>  16
<211>  23
<212>  RNA
<213>  Artificial

<220>
<223>  miARN

<400>  16
aaaagugcuu acagugcagg uag                                              23

<210>  17
<211>  18
<212>  RNA
<213>  Artificial

<220>
<223>  miARN

<400>  17
ugcuuccuuu cagagggu                                                    18

<210>  18
<211>  22
<212>  RNA
<213>  Artificial

<220>
<223>  miARN

<400>  18
uagcagcacg uaaauauugg cg                                               22

<210>  19
<211>  20
<212>  RNA
<213>  Artificial

<220>
<223>  miARN

<400>  19
cgugccaccc uuuuccccag                                                  20

<210>  20
<211>  19
<212>  RNA
<213>  Artificial

<220>
<223>  miARN

<400>  20
uggauuuuug gaucaggga                                                   19

<210>  21
<211>  22
<212>  RNA
<213>  Artificial

<220>
<223>  miARN

<400>  21
uguaaacauc cucgacugga ag                                                22


<210>  22
<211>  21
<212>  RNA
<213>  Artificial

<220>
<223>  miARN

<400>  22
aacauagagg aaauuccacg u                                                 21


<210>  23
<211>  23
<212>  RNA
<213>  Artificial

<220>
<223>  miARN

<400>  23
uuaaugcuaa ucgugauagg ggu                                               23


<210>  24
<211>  22
<212>  RNA
<213>  Artificial

<220>
<223>  miARN

<400>  24
ugagaacuga auuccauggg uu                                                22


<210>  25
<211>  23
<212>  RNA
<213>  Artificial

<220>
<223>  miARN

<400>  25
caacggaauc ccaaaagcag cug                                               23


<210>  26

<211>  22
<212>  RNA
<213>  Artificial

<220>
<223>  miARN

<400>  26
ucguaccgug aguaauaaug cg                                                22

## Description

La présente demande concerne des compositions et méthodes pour analyser et caractériser des patients atteints de cancer rectal ou du colon basées sur la détection de miARNs circulants. Elle concerne notamment des méthodes et réactifs (amorces, puces, DNA chips, sondes, etc.) pour déterminer ou évaluer l'évolution clinique de tels cancers, et plus particulièrement pour apprécier le caractère répondeur au traitement par radiochimiothérapie (RCT) néo-adjuvante des patients et/ou pour déterminer le risque de récidive métastatique chez ces derniers. L'invention est utilisable chez tout patient, et tout particulièrement chez les patients présentant un cancer du rectum localement avancé (stade II ou III).

### Introduction

Le cancer du rectum est le cinquième cancer le plus fréquent tous sexes confondus en France. Actuellement, plus de 17 000 cancers du rectum sont diagnostiqués chaque année en France et ce nombre devrait augmenter pour atteindre 45 000 nouveaux cas annuels à l'horizon 2020. Tous stades confondus, la probabilité de survie à 5 ans est d'environ 55% (Bossard N, 2007). La France fait partie des pays dans lesquels le risque de cancer du rectum est élevé, tout comme les autres pays d'Europe de l'Ouest, les USA, l'Australie et, également l'Asie. En effet, sur les 7 marchés majeurs (France, Japon, US, Allemagne, Italie, Espagne et le Royaume-Uni), 182 000 nouveaux cas de cancer du rectum ont été diagnostiqués en 2010 dont 40 000 aux USA. Dans ce contexte, tous les domaines de recherche apparaissent comme majeurs : dépistage, traitement, mais aussi prédiction de l'évolution clinique des patients.

Cependant, aucun marqueur performant n'existe à ce jour pour (i) permettre une détection précoce de la maladie, (ii) affiner le pronostic et guider ainsi la prise en charge des patients, et (iii) prédire la réponse au traitement pour adapter les thérapies.

Ainsi, les cancers du rectum sont classés en quatre stades : le stade I, qui est le moins avancé est essentiellement pris en charge par chirurgie, les stades II et III, pour lesquels les patients sont soumis à une radio-chimiothérapie combinée (RCT) préopératoire, et le stade IV, qui est un stade très avancé et métastasé, pour lequel la résection et des traitements alternatifs sont proposés. Lorsqu'un patient est diagnostiqué avec un cancer du rectum localement avancé (stade II ou III), il est typiquement traité par RCT avant la résection chirurgicale. Cependant, c'est seulement à l'issue du traitement que son efficacité est appréciée afin d'adapter le traitement du patient. Ainsi, il n'existe aujourd'hui aucune méthode fiable permettant, avant traitement RCT, de prédire la réponse du patient, ou son risque de récidive métastatique.

### Résumé de l'invention

La présente demande fournit des méthodes et compositions pour analyser et caractériser des patients atteints de cancer colorectal. Elle concerne plus particulièrement des méthodes et compositions pour prédire l'évolution clinique de patients atteints de cancer du rectum, notamment du cancer du rectum localement avancé, et notamment pour apprécier leur capacité à répondre à un traitement RCT et/ou le risque de récidive. L'invention découle de la mise au point et de la caractérisation de combinaisons de miARNs circulants permettant de caractériser ces cancers et d'identifier, avant traitement, les patients répondeurs et les patients à haut risque de récidive métastatique. L'invention est applicable en particulier à tout patient humain ayant un cancer du rectum, en particulier un cancer du rectum de stade II ou III, et permet d'adapter le traitement de manière optimisée et efficace.

Un objet de l'invention réside ainsi dans une méthode pour déterminer l'évolution clinique d'un patient atteint de cancer colorectal, de préférence d'un cancer du rectum, en particulier un cancer du rectum localement avancé, comprenant la détermination in vitro de la présence ou de la quantité d'un ensemble de miARNs circulants dans un échantillon biologique dudit patient, de préférence un échantillon de fluide biologique, ladite présence ou quantité étant indicative de l'évolution clinique du patient, ledit ensemble comprenant les miARNs suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

Dans un mode particulier, la méthode vise à déterminer si un patient atteint de cancer du rectum est susceptible de répondre à un traitement de radio et/ou chimio thérapie et/ou présente un risque de récidive métastatique. L'invention est tout particulièrement avantageuse pour déterminer si un patient atteint de cancer du rectum de stade II ou III est susceptible de répondre à un traitement de radiochimiothérapie combinée et/ou présente un risque de récidive métastatique.

Selon un premier mode de mise en oeuvre, l'invention vise une méthode pour déterminer si un patient atteint de cancer du rectum localement avancé (typiquement de stade II ou III) est susceptible de répondre à un traitement de radiochimiothérapie combinée, comprenant la détermination in vitro, dans un échantillon biologique dudit patient, de la présence ou quantité d'un ensemble de miARNs comprenant les miARNs hsa-miR-185, hsa-miR-376c, hsa-miR-106a, hsa-miR-25, hsa-miR-30a-5p, hsa-miR-16, hsa-let-7b et hsa-miR-19.

Selon un autre mode de mise en oeuvre, l'invention vise une méthode pour déterminer si un patient atteint de cancer du rectum localement avancé (typiquement de stade II ou III) est susceptible de répondre à un traitement de radiochimiothérapie combinée, comprenant la détermination in vitro, dans un échantillon biologique dudit patient, de préférence un échantillon de fluide biologique, de la présence ou quantité d'au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

Un autre objet de l'invention réside dans une méthode pour déterminer si un patient atteint de cancer du rectum localement avancé (typiquement de stade II ou III) est susceptible de récidiver après traitement, comprenant la détermination in vitro, dans un échantillon biologique dudit patient, de la présence ou quantité des 3 miARNs suivants : hsa-miR-30a-5p, hsa-miR-376c et hsa-miR-155.

La détection et/ou la quantification de miARNs peut être effectuée par toute technique connue en soi telle que notamment par hybridation, amplification, liaison à un ligand ou un test fonctionnel. Tout échantillon biologique peut être utilisé pour la mise en oeuvre de l'invention, de préférence un échantillon issu de fluide biologique, comme par exemple un échantillon de sang, plasma ou sérum.

Un autre objet de l'invention concerne une composition ou un dispositif (par exemple une puce, une plaque, un microarray, une colonne, etc.) comprenant un ensemble de sondes nucléiques, l'ensemble de sondes comprenant au moins une sonde nucléique complémentaire et spécifique de chaque miARN des ensembles suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux microARNs choisis hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

De préférence, la ou les sondes sont immobilisées sur un support. Dans un autre mode de mise en oeuvre, les sondes sont sous forme libre.

Un autre objet de l'invention concerne une composition comprenant une pluralité d'amorces nucléiques, ladite pluralité d'amorces comprenant une amorce nucléique ou une paire d'amorces nucléiques permettant l'amplification sélective d'une région au moins de chaque miARN des ensembles suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

Dans un mode particulier, les méthodes, dispositifs et/ou compositions de l'invention utilisent ou comprennent, outre des réactifs (e.g., sondes, amorces, ligands) spécifiques des ARNs caractéristiques de la réponse au traitement ou du risque de récidive d'un cancer du rectum, au moins un réactif (e.g., sonde, amorce, ligand) spécifique d'au moins un ARN contrôle (ou normalisateur), de préférence choisi parmi les ARN cités dans le Tableau 4. Les inventeurs ont en effet identifié plusieurs miARNs présentant un standard de déviation de leur expression entre les patients de la cohorte la plus faible possible et ayant ainsi une expression la plus constante possible entre patients. Ces miARNs représentent ainsi des marqueurs standards caractéristiques permettant une normalisation de la mesure chez les patients.

Un autre objet de la présente demande concerne un kit comprenant un compartiment ou conteneur comprenant au moins un, de préférence plusieurs, acides nucléiques comprenant une séquence complémentaire et/ou spécifique d'un ou plusieurs miARNs tels que définis précédemment et, optionnellement, des réactifs pour une réaction d'hybridation ou immunologique, ainsi que, le cas échéant, des contrôles et/ou instructions.

Un autre objet de l'invention concerne l'utilisation d'une composition ou d'un dispositif ou kit tel que défini ci-dessus pour la détermination de l'évolution clinique d'un patient ayant un cancer du rectum.

Un autre objet de l'invention réside dans une méthode de traitement d'un patient atteint d'un cancer du rectum, de préférence d'un cancer du rectum de stade II ou III, comprenant une première étape de détermination de la capacité du patient à répondre à un traitement tel que défini ci-dessus et, si le patient est répondeur, l'administration dudit traitement.

Un autre objet de l'invention concerne une amélioration aux méthodes de traitement du cancer du rectum chez un patient, l'amélioration consistant à mesurer la présence ou la quantité d'un ensemble de miARNs spécifiques tels que définis précédemment dans un échantillon dudit patient, avant et/ou pendant le traitement. La mesure permet d'adapter le traitement en fonction de l'évolution de la pathologie. Le traitement est typiquement un traitement radio- et/ou chimiothérapeutique.

L'invention est utilisable chez tout patient humain ayant un cancer du rectum, de préférence un cancer du rectum de stade II ou III, et permet la mise en place de stratégies thérapeutiques optimisées.

### Légende des Figures

Figure 1 : Courbe ROC de la prédiction de la signature par rapport à la réponse DWORAK.
Figure 2 : Courbes de survie des patients prédits sans récidives (vert) ou avec (rouge).

### Description détaillée de l'invention

La présente demande fournit des méthodes et compositions pour analyser et caractériser des patients atteints d'un cancer colorectal. Elle est particulièrement adaptée au cancer du rectum, mais peut également être utilisée pour le cancer du côlon. Elle concerne plus particulièrement des méthodes et compositions pour prédire l'évolution clinique de patients atteints d'un cancer du rectum, et plus particulièrement de cancer du rectum localement avancé de stade II ou III, et notamment pour apprécier leur capacité à répondre à un traitement RCT et/ou le risque de récidives métastatiques. L'invention repose sur la détermination ou le dosage de miARNs circulants particuliers permettant de caractériser ces cancers et d'identifier, avant traitement, les patients répondeurs et les patients à risque de récidives métastatiques.

La présente demande sera mieux comprise à l'aide des définitions suivantes :
Les microARNs (ou miARNs), sont impliqués dans les voies de régulation post-transcriptionnelle de l'expression des gènes. Les miARNs sont codés par le génome, puis transcrits en un précurseur sous forme d'une tige-boucle appelée pri-miARN. Des enzymes de la famille des RNAse en particulier de type III telles que DROSHA et DICER interviennent successivement pour cliver respectivement, le pri-miARN, puis pre-miARN en un petit fragment double-brin de 21 à 24 nucléotides de long. C'est la forme simple-brin qui est la forme mature et active. Cette forme permet de réguler l'expression des gènes en s'appariant à des ARN messagers portant une séquence complémentaire. Ceux-ci sont alors déstabilisés, dégradés, ou leur traduction est inhibée.

Le terme « microARN » ou « miARN » désigne au sens de l'invention des miARNs simple-brin matures, ainsi que leurs précurseurs et variants. Le terme microARN désigne également les transcrits primaires (pri-miARN) et précurseurs (pre-miARN) ainsi que les duplex miARN. Typiquement, l'invention mesure des présences ou quantités de miARNs matures.

Le terme miARN « circulant » désigne des miARNs présents dans un fluide biologique.

Le terme « détermination » inclut, au sens de l'invention, une prédiction, un pronostic, une évaluation ou une mesure, qualitative ou quantitative qui peut être absolue ou relative.

Le terme « RadioChimioThérapie combinée » ou « RCT » désigne un traitement combinant une irradiation et un traitement médicamenteux, plus préférentiellement une irradiation gamma et un anticancéreux. Le composé anticancéreux est préférentiellement choisi parmi la capécitabine, le 5-fluoro-uracile (5-FU), l'oxaliplatine, et leurs précurseurs ou analogues. Un traitement RCT typique comprend une irradiation gamma combinée à l'administration de capécitabine ou d'un précurseur ou analogue de celle-ci, éventuellement combinée à de l'oxaliplatine.

### Méthode de détermination des miARNs

La détection d'un miARN selon l'invention comprend la détection de la présence (ou non) de ce miARN ou, de préférence, une mesure de la quantité (relative ou absolue) d'un miARN. Des méthodes pour détecter ou mesurer la présence et quantité de miARNs sont connues en tant que tel dans l'art. Ces méthodes incluent, sans limitation, PCR quantitative, de la transcriptase inverse (RT-PCR), l'hybridation avec des sondes spécifiques, par northern blot, affinités ou similaires.

Dans une réalisation particulière, les miARNs sont détectés ou mesurés dans un échantillon par hybridation, amplification, ligands ou un test fonctionnel. Dans une réalisation particulière, une partie aliquote d'un échantillon biologique est mise en contact avec une sonde d'acide nucléique, une amorce d'acide nucléique ou un ligand, caractéristiques d'au moins un miARN cible, et la présence ou la quantité de complexes formés entre ladite sonde, amorce, ou ligand et acides nucléiques dans l'aliquote est déterminée. Dans ces méthodes, la sonde ou le ligand peut être en solution ou immobilisé sur un support, comme un « microarray ». Aussi, l'échantillon biologique peut être traité avant la détermination, par exemple, dilué ou concentré ou enrichi pour les miARNs. Le miARN dans l'échantillon peut également être étiqueté afin de faciliter la détermination.

Dans une réalisation particulière, la méthode comprend (i) éventuellement le marquage ou étiquetage des miARNs circulants présents dans un échantillon biologique, et (ii) l'hybridation desdits miARNs (éventuellement marqués/étiquetés) sur un support (par exemple un microarray) comprenant au moins un acide nucléique sonde spécifique pour un miARN cible pour obtenir un profil d'hybridation. Le profil d'hybridation étant indicatif de l'évolution clinique du cancer chez le sujet.

Dans un autre mode de réalisation particulier, la méthode comprend (i) l'obtention des miARNs circulants à partir d'un échantillon biologique et (ii) la mise en contact des miARNs circulants avec un ensemble d'au moins deux, de préférence au moins trois, amorces d'acide nucléique, lesdites au moins deux, préférablement au moins trois amorces d'acide nucléique permettant d'amplifier spécifiquement un miARN cible, afin d'obtenir un produit d'amplification, le produit d'amplification étant indicatif de l'évolution clinique du cancer dans le sujet.

Dans un mode de réalisation préféré, les miARNs sont déterminés par PCR en temps réel utilisant des compositions d'amorces spécifiques selon l'invention.

Dans un autre mode de réalisation, les miARNs sont déterminés par analyse microarray, c'est-à-dire, en mettant en contact un échantillon test avec un « microarray » comprenant une pluralité de sondes spécifiques immobilisées sur sa surface, permettant ainsi la réaction d'hybridation se produire et l'analyse du profil de l'hybridation.

Pour analyser les miARNs circulants, la première étape consiste à obtenir et/ou préparer un échantillon biologique. L'échantillon test peut-être être obtenu auprès de n'importe quel échantillon biologique qui contient des miARNs circulants. Dans une réalisation particulière, l'échantillon test est ou provient d'un fluide biologique tel que, sans limitation, sang, plasma, sérum, salive, urine, liquide céphalo-rachidien, liquide de lavage bronchique ou liquide de lavage de sinus. D'autres fluides peuvent également être utilisés pour exécuter l'invention comme la moelle osseuse, cervicale, vaginale, vésico-urétral, anale, gorge, gingivale, ou écouvillon oculaire, ganglions, humeur aqueuse, liquide amniotique, cérumen, le lait maternel, sperme, liquide prostatique, éjaculat féminin, sueur, larmes, liquide du kyste, liquide pleural ou péritonéal, liquide péricardique, liquide interstitiel, menses, pus, sébum, sécrétions vaginales, sécrétion muqueuse, ponction broncho-pulmonaire ou sang de cordon ombilical. D'autres échantillons comprennent des échantillons de tissus, biopsie, résection de tumeur ou noeuds.

Différentes méthodes existent pour l'obtention et la préparation des liquides biologiques comme le sang, sérum ou échantillons de plasma. En outre, les tubes de prélèvement sanguin sont disponibles dans le commerce auprès de nombreuses sources. Un échantillon préféré est le sang, le sérum ou le plasma, tels que plasma pauvre en plaquettes. De préférence, pour analyser les miARNs circulants, l'échantillon est collecté et traité au cours des premières 1 à 24 heures pour minimiser la dégradation des miARNs en circulation et minimiser la libération des miARNs de cellules intactes dans l'échantillon. L'échantillon (p. ex., sang, plasma, sérum, urine, salive et autres) peut être congelé et traité par la suite. De préférence, avant de déterminer les taux circulants de miARNs, l'échantillon est traité. Le traitement peut-être être réalisé pour normaliser les miARNs, diluer ou concentrer l'échantillon, enrichir en miARNs, marquer ou étiqueter les miARNs, supprimer les cellules ou les autres fractions, protéger les ARNs (par exemple, inhiber la RNAse), etc.

Dans une réalisation particulière, la méthode comprend :
a) fournir un échantillon biologique prélevé d'un sujet, l'échantillon comprenant des miARNs circulants,
b) traiter l'échantillon par dilution, concentration, enrichissement en miARNs, protection des ARNs, ou pour enlever des cellules,
c) éventuellement congeler l'échantillon, et
d) déterminer des miARNs circulants dans l'échantillon.

Les étapes b) et c) peuvent être inversées. Dans un tel cas, l'échantillon est décongelé avant l'étape de traitement. Les miARNs circulants peuvent-être être extraits ou partiellement purifiés à partir de l'échantillon biologique avant la détermination. À cette fin, l'ARN circulant total peut être purifié par homogénéisation en présence d'un tampon d'extraction d'acides nucléiques, suivi par une centrifugation. Les molécules d'ARN peuvent être séparées par électrophorèse sur gel d'agarose suivant les techniques habituelles. Les kits sont disponibles dans le commerce pour préparer les miARNs provenant d'échantillons biologiques.

Pour déterminer un miARN sélectionné par hybridation, une ou plusieurs sondes appropriées spécifiques pour ledit miARN peuvent être produites à l'aide de la séquence nucléotidique dudit miARN. Les séquences d'acides nucléiques des miARNs sont tirées des bases de données «miRBase::Sequences» de la Wellcome Trust Sanger Institute (http://microma.sangetac.uk/sequences/index.shtml) et miRBase httµ://www.mirbase.org/.

Les sondes préférées sont des molécules d'acide nucléique simple brin de 10 à 500 bases de longueur, plus préférentiellement de 10-200. Les sondes particulièrement préférées ont une longueur semblable à celle de la cible miARN. Elles contiennent une séquence complémentaire à la cible miARN, préférablement une séquence qui est parfaitement complémentaire. Dans certains modes de réalisation, un certain degré de mésappariement peut être toléré aussi longtemps que la sonde peut s'hybrider spécifiquement à la cible miARN dans un échantillon complexe, lorsqu'il est placé dans des conditions stringentes.

Des méthodes pour marquer/étiqueter des sondes d'ADN ou d'ARN, ainsi que les conditions pour l'hybridation des acides nucléiques cible sont connues en tant que tel dans l'art, tel que décrit par exemple, dans le clonage moléculaire: un manuel de laboratoire, J. Scott et al., eds., 2e édition, Cold Spring Harbor Laboratory Press, 1989, qui y sont incorporés par référence.

Dans un mode de réalisation préféré de l'invention, le miARN dans un échantillon est déterminé par amplification spécifique. Diverses techniques existent pour amplifier les séquences d'acides nucléiques de miARNs, comme par exemple la transcription inverse (RT), réaction en chaîne par polymérase (PCR), PCR en temps réel (PCR quantitative (q-PCR)), amplification de base de la séquence des acides nucléiques, amplification multiplex sonde ligatable, amplification de cercle roulant ou amplification de déplacement de brin. Dans un mode de réalisation préféré, la détermination est faite par transcription inverse des miARNs, suivie de l'amplification du transcrit inverse par polymerase chain reaction (RT-PCR). L'amplification utilise généralement des amorces d'acide nucléique. Une amorce est généralement environ 15 à 40 nucléotides de long, simple brin et contient une région (de préférence parfaitement) complémentaire à une partie de la cible miARN. Une paire d'amorces est généralement utilisée comprenant : une amorce sens, qui peut s'apparier à la cible miARN, et une amorce anti-sens, qui sert à s'apparier à l'ensemble de la cible de transcription inverse miARN. En règle générale, le niveau du miARN circulant mesuré est comparé à une valeur de contrôle ou de référence pour déterminer si le niveau est réduit ou augmenté. Le contrôle ou la référence peut être un contrôle externe, comme un miARN circulant dans un échantillon d'un sujet de contrôle. Le contrôle externe peut être une quantité connue d'un ARN de synthèse. Le contrôle peut être un échantillon mis en commun, moyen ou individuel. La valeur de contrôle ou de référence peut être une valeur moyenne ou médiane de référence pour un miARN donné dans une situation de référence. En outre, dans un mode de réalisation préféré, la méthode comprend la détermination d'au moins un miARN contrôle, tel qu'énuméré dans le tableau 4, permettant la normalisation des résultats. Dans certains modes de réalisation, il est souhaitable de déterminer simultanément le niveau d'expression d'une pluralité de miARNs circulant différents dans l'échantillon. Dans certains cas, il est peut-être même souhaitable de déterminer le niveau d'expression de tous les miARNs connus. L'évaluation des niveaux d'expression pour plusieurs miARNs circulants peut être réalisée en utilisant l'amplification ou microarrays comprenant une pluralité de sondes ou en utilisant une combinaison de différentes amorces. L'utilisation de puces ou amorces présente de nombreux avantages pour la détection de miARNs. En effet, plusieurs centaines de miARNs sont reconnaissables à la fois à un point dans le temps. En outre, une petite quantité d'ARN est nécessaire. L'évaluation des niveaux d'expression pour plusieurs miARNs circulants peut-être être effectuée à l'aide de tige-boucles spécifiques RT suivie d'une PCR quantitative, particulièrement faible densité RT-qPCR comme TLDA (TaqMan ® faible densité tableaux ; Life Technologies). Comme indiqué dans la section expérimentale, cette méthode peut être utilisée efficacement pour évaluer simultanément un grand nombre de miARNs dans un échantillon complexe. La méthode peut donc comprendre la détermination d'au moins 2, 3, 4, 5, 10, 15, 20, 30, 40, 50 ou même plusieurs centaines de miARNs circulants.

### Identification de combinaisons de miARNs circulants caractéristiques de l'évolution clinique de cancers du rectum

La présente demande décrit des combinaisons de miARNs circulants dont la détection permet de déterminer l'évolution clinique de cancers du rectum, plus particulièrement de cancers du rectum localement avancés. L'invention fournit en particulier des combinaisons de miARNs circulants prédictifs de l'efficacité de la radiochimiothérapie (RCT) préopératoire et pronostiques de la survie sans récidive métastatique. Ces ensembles de miARNs sont particulièrement avantageux car ils renseignent sur le profil des cancers avant tout traitement et permettent de définir un protocole thérapeutique optimisé.

Un objet particulier de l'invention réside ainsi dans une méthode pour déterminer si un patient atteint de cancer du rectum localement avancé est susceptible de répondre à un traitement de radiochimiothérapie combinée, comprenant la détermination in vitro, dans un échantillon biologique dudit patient, de la présence, absence, ou quantité des miARNs suivants : hsa-miR-185, hsa-miR-376c, hsa-miR-106a, hsa-miR-25, hsa-miR-30a-5p, hsa-miR-16, hsa-let-7b et hsa-miR-19.

Le tableau 1 présente les caractéristiques d'expression, les références et les séquences de chacun de ces miARNs.

Dans un autre mode particulier, la méthode comprend la détermination de la présence, absence, ou quantité d'au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b..

Le tableau 2 présente les caractéristiques d'expression, les références et les séquences de chacun de ces miARNs.

Dans une variante de ce mode, l'invention comprend la détermination d'au moins 3 miARNs distincts cités ci-dessus, encore plus particulièrement d'au moins 4, au moins 5, au moins 6, au moins 7, au moins 8, au moins 9, au moins 10, au moins 11, au moins 12, au moins 13 ou de tous lesdits 14 miARNs.

A cet égard, un exemple particulier de mise en oeuvre comprend la détermination de la présence, absence ou quantité d'au moins hsa-miR-17-5p et d'un ARN choisi parmi hsa-miR-16-5p, hsa-miR-195-5p, hsa-miR-571 et hsa-miR-1227-3p.

Un autre exemple particulier de mise en oeuvre comprend la détermination de la présence, absence ou quantité d'au moins hsa-miR-16-5p et d'un ARN choisi parmi hsa-miR-195-5p, et hsa-miR-1227-3p.

Des méthodes particulières utilisent au moins les miARNs suivants :
. hsa-miR-17-5p et hsa-miR-571,
. hsa-miR-17-5p et hsa-miR-571 et mmu-miR-451a,
. hsa-miR-17-5p et hsa-miR-195-5p,
. hsa-miR-17-5p et hsa-miR-16-5p,
. hsa-miR-16-5p et hsa-miR-195-5p,
. hsa-miR-16-5p et hsa-miR-1227-3p,
. hsa-miR-17-5p et hsa-miR-571 et mmu-miR-451a et hsa-miR-16-5p et hsa-miR-195-5p et hsa-miR-516b-3p, ou
. hsa-miR-17-5p, hsa-miR-571, mmu-miR-451a, hsa-miR-16-5p, hsa-miR-195-5p, hsa-miR-516b-3p, hsa-miR-1290 et hsa-miR-1227-3p.

Un autre objet de l'invention réside dans une méthode pour déterminer si un patient atteint de cancer du rectum localement avancé (typiquement de stade II ou III) est susceptible de récidiver après traitement, comprenant la détermination in vitro, dans un échantillon biologique dudit patient, de la présence ou quantité des miARNs suivants : hsa-miR-30a-5p, hsa-miR-376c et hsa-miR-155.

Le tableau 3 présente les caractéristiques d'expression, les références et les séquences de chacun de ces miARNs.

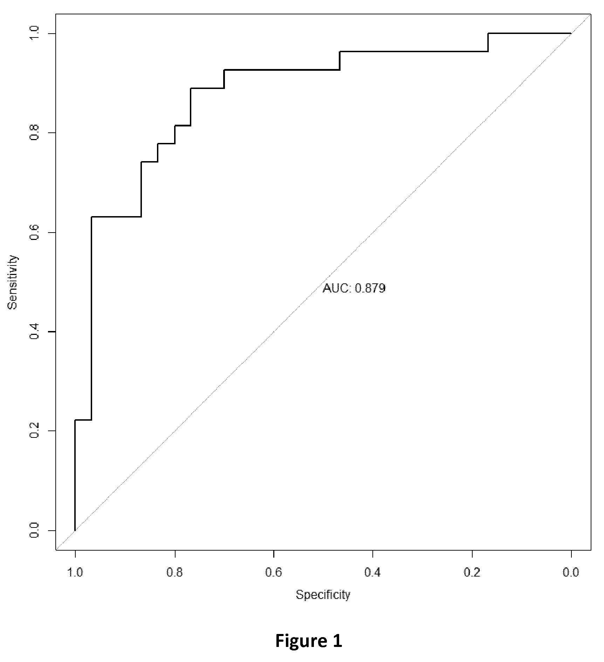

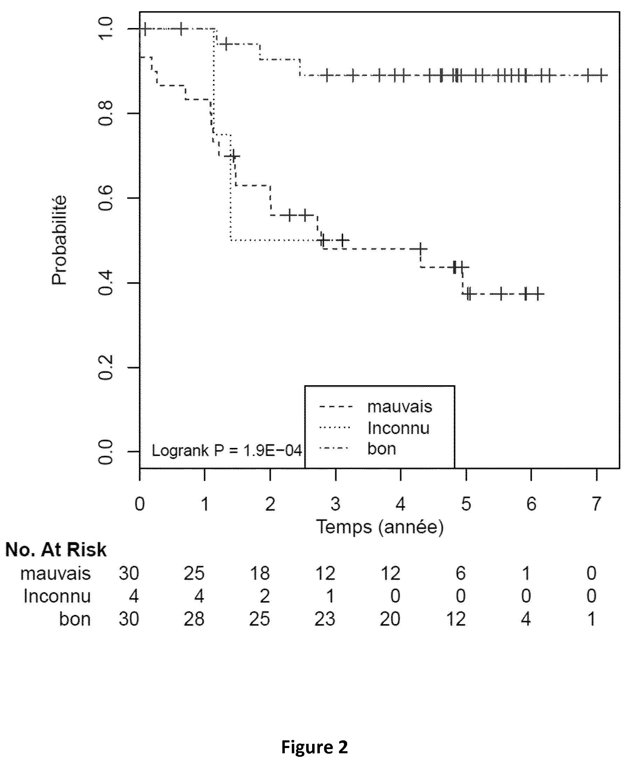

Comme illustré dans la section expérimentale, les combinaisons de miARNs de l'invention permettent de déterminer avec précision et fiabilité le potentiel d'efficacité d'un traitement RCT chez des patients ayant un cancer du rectum ou le risque de récidive. Les combinaisons de miARNs décrites dans la présente demande permettent notamment de prédire, avant tout traitement du patient, la réponse au traitement avec une sensibilité et spécificité très élevées, supérieures à 0,75 (voir Figure 1), et de guider le praticien dans la prédiction de la récidive métastatique des patients (voir Figure 2). Les méthodes de l'invention se distinguent des approches de l'art antérieur notamment par:
(i) une orientation thérapeutique personnalisée des patients dès leur prise en charge (avant tout traitement).
   Les résultats de l'analyse du premier bilan sanguin des patients permettent au praticien de discriminer les patients répondeurs des non répondeurs ainsi que ceux qui peuvent être sujet à la récidive. Les patients répondeurs et sans risque de récidive peuvent être orientés vers une désescalade thérapeutique. Les patients répondeurs et avec récidive ou non-répondeurs et/ou avec récidive, quant à eux, peuvent rapidement être orientés vers des protocoles ciblés.
(ii) un confort et une durée de vie améliorée pour les patients. Les analyses sont non invasives car réalisées à partir d'un prélèvement sanguin classique (par exemple du sérum) et non à partir d'une pièce chirurgicale. L'identification précoce du statut répondeur et sans récidive de certains patients permet aussi au praticien de procéder à un allégement thérapeutique ce qui améliore significativement les conditions de vie des patients. Les patients des autres statuts, quant à eux, peuvent rapidement être orientés vers des protocoles adaptés, ce qui augmente les chances de rémission.
(iii) une précision de l'analyse. Le traitement néo-adjuvant entraîne une diminution de la taille et du stade de la tumeur rectale. Pour cette évaluation trois termes sont couramment employés lorsque la réponse au traitement par radiochimiothérapie des cancers du rectum est étudiée : le down staging, le down sizing, et la régression tumorale histologique. La régression histologique repose sur une analyse détaillée de la pièce chirurgicale et à l'heure actuelle, de nombreux systèmes de graduation histologique basés sur la densité cellulaire tumorale résiduelle ont été proposés. Le grade selon la méthode de DWORAK (Dworak et al., int. J Colorectal dis., 1997) qui regroupe les patients en 5 classes est couramment utilisé depuis de nombreuses années par les centres nationaux et internationaux. Cette graduation permet une bonne identification des deux extrêmes, à savoir les très bons répondeurs et les très mauvais répondeurs. Cependant, les cas intermédiaires représentant 20 à 30% des patients ne sont pas correctement classés. L'invention permet aussi une classification précise des patients au statut actuellement non clairement défini. Par ailleurs, bien que l'IRM soit actuellement la modalité la plus évaluée comme alternative dans la prédiction de la réponse au traitement néoadjuvant par radiochimiothérapie (Molinari C et al Clin Colorectal Cancer 2015 ; Beets tan RG et al. Nat Rev Gastroenterol Hepatol 2014), cette technique n'est pas validée en pratique courante et il n'y a pas encore de consensus sur le score de réponse reconnu et validé prospectivement.
(iv) un temps d'analyse réduit. A l'heure actuelle, l'analyse de la pièce chirurgicale requiert un échantillonnage exhaustif avec des étapes pré analytiques de fixation, inclusion en paraffine et réalisation de coupes. Par la suite, l'analyse minutieuse sous microscope par un anatomopathologiste expert est nécessaire pour une interprétation optimale. La réalisation de chacune de ces étapes est fastidieuse et chronophage comparée à la méthode de l'invention, qui peut être réalisée d'un point de vue pratique en 4h par du personnel technique de laboratoire sans formation spécifique additionnelle. L'analyse des résultats du test est de quelques minutes seulement et peut être réalisée facilement moyennant l'utilisation d'un algorithme dédié.
(v) un coût sociétal réduit. L'orientation précoce des patients vers un traitement adapté augmentera significativement la réussite du traitement et diminuera les situations d'échec qui signifient une ré-orientation vers de nouveaux protocoles qui sont des situations difficiles pour les patients et coûteuses pour l'organisme de remboursement. Le coût modéré de la méthode de l'invention constitue un atout supplémentaire pour l'organisme de remboursement.

Dans un mode particulier, l'invention comprend (i) l'obtention d'un échantillon de fluide biologique test, (ii) éventuellement le traitement de l'échantillon test pour éliminer les cellules éventuellement présentes et/ou enrichir les acides nucléiques, par exemple, et (iii) la détermination de la présence ou quantité des ensembles de miARNs tels que définis ci-avant.

Dans un mode particulier, la méthode comprend en outre la détermination d'au moins un miARN de référence (ou normalisateur), choisi de préférence parmi hsa-miR-146a-5p, hsa-miR-191-5p et/ou hsa-miR-126-3p décrits dans le tableau 4. La présente invention montre en effet que ces miARNs sont très peu modulés dans les différentes cohortes étudiées, et permettent donc de normaliser les résultats sur les marqueurs. Avantageusement, la détermination de la présence, quantité ou absence des miARNs marqueurs comprend ainsi une étape de normalisation par rapport à la quantité mesurée de l'un ou de plusieurs des trois miARNs de référence ci-dessus.

Un autre objet de l'invention concerne une composition ou un dispositif (par exemple une puce, une plaque, un microarray, une colonne, etc.) comprenant un ensemble de sondes nucléiques, l'ensemble de sondes comprenant au moins une sonde nucléique complémentaire et spécifique de chaque miARN des ensembles de miARNs suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

Dans les dispositifs de l'invention, les sondes sont préférablement immobilisées sur une surface d'un matériau support, généralement selon un arrangement ordonné. Le matériau support peut être solide ou semi-solide et présenter au moins une surface, plane ou non, permettant l'immobilisation de sondes nucléiques. De tels supports sont par exemple une lame, bille, membrane, filtre, colonne, plaque, etc. Ils peuvent être réalisés en tout matériau compatible, comme notamment du verre, silice, plastique, fibre, métal, polymère, etc. Les réactifs peuvent être immobilisés sur la surface du support par des techniques connues, ou, dans le cas des acides nucléiques, synthétisés directement in situ sur le support. Des techniques d'immobilisation incluent l'adsorption passive (Inouye et al., J. Clin. Microbiol. 28 (1990) 1469), la liaison covalente. Des techniques sont décrites par exemple dans WO90/03382, WO99/46403. Les réactifs immobilisés sur le support peuvent être ordonnés selon un schéma pré-établi, pour faciliter la détection et l'identification des complexes formés, et selon une densité variable et adaptable.

Un autre objet de l'invention concerne une composition comprenant une pluralité d'amorces nucléiques, ladite pluralité d'amorces comprenant une amorce nucléique permettant l'amplification sélective d'une région au moins de chaque microARN des ensembles suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux microARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

Dans un mode de réalisation préféré, la composition comprend (i) au moins les amorces qui amplifient spécifiquement au moins une région des miARNs circulant formant les signatures définies dans l'un des tableaux 1 à 3 et (ii) au moins une amorce qui amplifie spécifiquement au moins un miARN normalisateur tel que listé dans le tableau 4. Ces compositions sont particulièrement adaptées pour la surveillance ou l'analyse du cancer du rectum par amplification PCR, en particulier par PCR en temps réel.

La présente invention se rapporte en outre à un kit contenant un dispositif ou une composition tel que défini ci-dessus. Le kit peut contenir en outre un ou des réactifs pour une réaction d'hybridation ou d'amplification, un témoin ou un manuel d'instructions, etc.

La présente invention se rapporte également à un kit contenant une série d'amorces d'acides nucléiques tels que définis ci-dessus. Le kit peut contenir en outre des réactifs pour une réaction d'amplification, un témoin ou un manuel d'instructions, etc.

L'invention porte également sur l'utilisation de sondes, amorces, dispositifs, compositions ou kits tels que définis ci-dessus pour la détermination in vitro la réactivité RCT ou du risque de récidive d'un cancer du rectum chez un sujet.

D'autres aspects et avantages de l'invention seront décrits dans les exemples suivants, qui sont illustratifs de l'invention.

### Exemples

### Matériels et méthodes

### Description de la cohorte des patients

La cohorte utilisée compte 69 patients atteints de cancer du rectum. Pour chaque patient, le sérum a été prélevé avant tout type de traitement. La base clinico-biopathologique pour les patients de l'étude comprend : (i) les données d'identification (sexe, âge au diagnostic), (ii) le statut pré-thérapeutique (uTNM), la caractérisation de la pièce de résection chirurgicale (histologie, ypTN, régression tumorale selon Dworak) et le suivi (traitement adjuvant, récidive locale ou métastatique, état du patient).

Les principales caractéristiques de la cohorte sont décrites dans le tableau 6.

La cohorte étudiée est composée de patients atteints d'un cancer du rectum de stade II à stade III. Les sera utilisés pour le profilage des miARNs ont été prélevés avant tout type de traitement. Les patients de la cohorte ont été pris en charge selon les standards européen actuels, qui sont également suivis hors zone Europe, et qui comprennent (i), un traitement néo-adjuvant incluant une radiothérapie (RT) avec 45Gy délivrée sur 5 semaines associée à une chimiothérapie à base de capécitabine et d'oxaliplatine ou leurs analogues, et (ii) résection chirurgicale dans les 6 à 8 semaines suivant la fin du traitement. A partir de la pièce chirurgicale, une analyse histologique est réalisée et la régression tumorale évaluée selon la classification de *DWORAK.* Elle a pour objet d'évaluer la réponse des patients au traitement néo-adjuvant en les classant comme non répondeurs s'ils ont un score de Dworak de 0 ou 1, ou répondeurs s'ils ont un score de Dworak 3 ou 4. Les réponses intermédiaires correspondent à des tumeurs avec un score de Dworak 2 et représentent 26 % des patients de la cohorte étudiée. Selon la réponse des patients, un traitement adjuvant à base de chimiothérapie a été prescrit aux patients de la cohorte étudiée.

Pour ces mêmes patients une IRM est réalisée avant tout traitement, puis après les 6 à 11 semaines suivant la fin du traitement. La différence entre les deux clichés permet de définir des répondeurs s'ils ont un score d'évolution de 1 ou 2, et permet de définir des non-répondeurs s'ils ont un score d'évolution de 0.

Le suivi médian des patients de cette cohorte rétrospective est à plus de 5 ans, ce qui a permis d'observer que 5 % des patients récidivent localement et 30 à 35 % des patients récidivent à distance. Ces résultats sont en concordance avec les observations qui ont été publiées par différents centres nationaux et internationaux (Gérard JP et al. J Clin Oncol. 2006;24:4620-5 ; Bosset JF et al., N Engl J Med 2006; 355:1114-1123).

En parallèle une classification par la réponse IRM qui regroupe en 0 les non-répondeurs et en 1, 2 les répondeurs a été réalisée. La cohorte comprend cette fois-ci 46 individus divisés en 50/50 soit 23 individus par classe.

### Evaluation de la réponse au traitement d'induction selon la classification de DWORAK

La réponse au traitement néo-adjuvant de RCT a été évaluée par analyse histologique de la pièce de résection chirurgicale. La régression tumorale a été analysée de manière semi-quantitative en appréciant à la fois le pourcentage de cellules viables et le pourcentage de territoires fibreux ou colloïdes. La classification de DWORAK en 5 groupes de réponse (scores de 0 à 4) a été retenue (Dworak O et al,. 1997, Int J. Colorectal Dis.) :
(0) pas de régression
(1) tumeur majoritaire avec fibrose et/ou mucine
(2) fibrose ou mucine majoritaire, quelques cellules ou structures tumorales
(3) très rares cellules tumorales avec fibrose et/ou mucine majoritaire
(4) réponse complète, absence de cellules tumorales; fibrose et/ou mucine uniquement

Pour les analyses statistiques de la réponse au traitement, les patients de Dworak 0, 1 et 2 ont été regroupés dans le groupe des non répondeurs et les patients Dworak 3 et 4 dans le groupe des répondeurs.

### Evaluation de la réponse au traitement d'induction selon la réponse volumétrique en IRM

Une image IRM est réalisée avant traitement néo-adjuvant puis 6 à 11 semaines après le traitement. La réponse IRM établit la différence entre les deux clichés.
(0) réponse insuffisante en volume
(1) Bonne réponse en volume (Diminution > 70%)
(2) Réponse complète
Nougaret et al. (Radiology. 2012 May;263(2):409-18 )

Pour les analyses statistiques de la réponse au traitement, les patients avec réponse IRM 0 ont été regroupés dans le groupe des non répondeurs et les patients avec réponse IRM 1 et 2 dans le groupe des répondeurs.

### Analyse de la survie sans métastases

L'analyse de la survie sans métastase est l'évènement de la survenue de métastases. Les taux de survie sont définies en considérant le délai depuis la date de chirurgie jusqu'à la date de survenue de l'évènement. Les patients vivants sans métastase sont censurés à la date des dernières nouvelles.

### Extraction et purification des miARNs

L'extraction des miARNs a été réalisée avec le kit Macherey-Nagel (NucleoSpin miARN Plasma).

### Profilage des miARNs par Taqman Low Density Arrays

Les plaques TLDA A et B possèdent 384 sondes qui permettent de détecter simultanément environ 760 miARNs par real-time PCR.

### Validation technique de l'expression des miARNs candidats réalisée par qPCR single

Les miARNs identifiés comme les plus robustes statistiquement suite au profilage par TLDA commercialisés la société Lifetechnologies ont été validés techniquement par Single qPCR. Cette étape consiste en la confirmation de l'expression des miARNs candidats par l'utilisation de plaques à façon fabriquées par la société Lifetechnologies et *coated* avec des sondes et amorces de rétrotranscription et d'amplification de référence identique à celles qui ont été précédemment utilisées pour le profilage.

### Logiciels et R scripts utilisés

Les résultats sont obtenus à partir d'une plateforme développée en interne qui se base sur le logiciel R (www.r-project.org). Cette première et STATA (Data Analysis and Statistical Software, StataCorp LP) ont été utilisés conjointement pour le prétraitement des données et l'analyse statistique

### Filtrage des données

Les données sont filtrées en fonction des données de contrôle/qualité du logiciel GES version: 1.0.3. Ces paramètres permettent d'exclure les valeurs des miARNs qui résultent d'effet technique et non biologique. Les critères de qualité ont été affinés par les données constructeur et l'expertises acquises dans le domaine. Elles concernent trois valeurs : le score d'amplification, le critère de confiance de la qualité et la valeur de de cycle obtenue.

### Identification de miARNs de référence

Logiciel GeNorm (Vandesompele J et al., Genome Biol 2002, 3:0034.1-0034.11) permet d'estimer la variation par paires (déviation standard (SD) du ratio d'expression transformée par logarithme) d'un miARN contrôle « détecteur » donné avec tous les autres miARNs contrôles de l'expérience. De ceci, une mesure de stabilité (M) est calculée comme la moyenne de la variation de la paire. M est défini comme le SD du log du ratio d'expression de la paire de miARNs détecteur. En assumant que la paire n'est pas co-régulée, la paire de détecteurs avec la valeur de M la plus faible est celle qui a l'expression la plus stable dans l'ensemble des échantillons.

A partir des données brutes du profilage par TLDA, les miARNs miR-146a, miR-126 et le miR-191 ont été sélectionnés, qui possèdent les scores M-value les plus robustes. Ces miARNs ont été sélectionnés pour les normalisations des données brutes des plaques TLDA (profilage) et à façon (validation technique) pour la réponse au traitement et la récidive métastatique (voir Tableau 4).

L'algorithme de GeNorm est implémenté dans le package R «HTqPCR» (v2.13, auteurs Heidi Dvinge et Paul Bertone, EMBL-EBI, http://www.ebi.ac.uk/research/bertone/software).

Normalisations des données brutes :
La normalisation a été réalisée selon la méthode deltaCt à partir de la moyenne des Cts des miARNs de référence identifiés.

Analyses univariées et multivariées de la réponse au traitement :
Les logiciels et approches utilisées pour ces analyses sont :
   (i) Package R « samr » Significance Analysis of Microarrays R (v2.00, auteurs R. Tibshirani, G. Chu, Balasubramanian Narasimhan, Jun Li, Standford, http://statweb.stanford.edu/~tibs/SAM). Permet de sélectionner les facteurs différentiellements exprimés entre les classes de patients répondeurs/non répondeurs comme définis ci-dessus, ce test permet également de gérer le problème de la multiplicité des tests.
   (ii) Le test de Kruskal-Wallis utilisé sous R
   (iii) Les analyses multivariées de la réponse ont été réalisées suivant un modèle de régression logistique multivariée avec le package R Glmnet.
   (iv) Les courbes ROC ont été générées à partir du package R « risksetROC ».

### Analyses univariées et multivariées de la survie sans métastases

Les courbes de survie ont été estimées par la méthode de Kaplan-Meier et comparées avec un test du logrank. Pour les analyses univariées, une p-value non ajustée du test du logrank a été calculée afin de prendre en compte la multiplicité des tests. Le Package R utilisé est « Survival » (Modeling Survival Data: Extending the Cox Model. Terry M. Therneau and Patricia M. Grambsch (2000) Springer, New York. ISBN 0-387-98784-3) qui permet l'analyse en univariée et multivariée (régression de Cox).

### Résultats

### Détermination d'une signature miARNs prédictive de la réponse à la radiochimiothérapie néoadjuvante en classification Dworak

Après normalisation avec les miARNs de référence préalablement définis, les analyses réalisées à partir des séra de patients répondeurs (Dworak 3 et 4) et de patients non répondeurs (Dworak 0, 1 et 2) ont permis d'établir une signature de miARNs particulièrement prédictive. La signature est définie initialement par régression logistique, et elle est composée des miARNs dont la p-valeur est significative (< 0.05) lors de la régression logistique. Ladite signature est alors appliquée sur les données obtenues pour évaluer sa capacité prédictive à travers la sensibilité -les répondeurs bien prédits sur l'ensemble des répondeurs- et la spécificité -les non répondeurs prédits sur l'ensemble des non répondeurs.

La composition de la signature est présentée dans le Tableau 1. Ses capacités prédictives sont données dans le Tableau 5 et sur la figure 1.

Comme indiqué, cette signature comprend 8 miARNs distincts. Sur la cohorte testée, sa spécificité et sa sensibilité sont de 0.8 et 0.78, respectivement. La figure 1 montre une aire sous la courbe ROC de 0.79, qui caractérise l'excellente performance du test.

### Détermination d'une signature miARNs prédictive de la réponse à la radiochimiothérapie néoadjuvante en classification par IRM

Après normalisation avec les miARNs de référence préalablement définis, les analyses réalisées à partir des séra de patients répondeurs (IRM 1 et 2) et de patients non répondeurs (IRM 0) ont permis de sélectionner des miARNs présentant un signal significatif. Ces miARNs sont résumés dans le Tableau 2.

Ledit signal est considéré comme significatif lorsque la p-valeur du test de Wilcoxon Mann Whitney est inférieure à 0.1. L'utilisation de ce test est justifiée d'une part car la réponse au traitement est dichotomisée en Répondeur, Non-répondeur et d'autre part la distribution de certains miARNs.

Comme indiqué dans le tableau 2, les marqueurs identifiés présentent tous une p-valeur très significative et permettent, en combinaisons de 2 ou plus, d'obtenir une spécificité et une sensibilité prédictives très élevées.

### Détermination d'une signature miARNs prédictive du risque de récidive métastatique

Après normalisation avec les miARNs de référence préalablement définis, les analyses réalisées à partir des séra de patients récidivistes et non récidivistes ont permis d'établir une signature de miARNs particulièrement prédictive.

A l'instar de la détermination d'une signature pour la réponse au traitement réponse à la radiochimiothérapie néoadjuvante en classification Dworak, un modèle de régression a été utilisé. La récidive étant sujette à la censure des données (impossibilité de savoir si le patient fait une récidive métastatique suite au décès ou à l'absence de suivi de celui-ci), le modèle de Cox est utilisé. La signature est composée des miARNs dont la p-valeur est significative (< 0.05) lors de la régression du modèle de Cox.

Ladite signature est alors appliquée sur les données obtenues pour évaluer sa capacité prédictive.

La composition de la signature est présentée dans le Tableau 3. Ses capacités prédictives sont démontrées dans la figure 2.

Comme indiqué, cette signature comprend 3 miARNs distincts. Sur la cohorte testée, les patients en l'absence de prédiction ont une probabilité de ne pas faire de récidive de 65% au cours des 7 années qui suivent le traitement. Comme le montre la Figure 2 , les patients prédits comme sans risque de récidive ont une probabilité de ne pas faire de récidive de 90%. A l'inverse les patients prédits avec un risque de récidive ont une probabilité de ne pas faire une récidive de 35%. Le résultat obtenu est significatif comme l'atteste la p valeur du logrank largement inférieure à 0.05.

### Composition et kit prédictifs du risque de récidive métastatique

La préparation d'un kit contenant des amorces spécifiques de la récidive comprend la préparation d'un support de RT pour la transcription reverse et d'un support PCR pour l'amplification.

Le support contient les contrôles positifs (sérum contrôle) et négatifs (tube d'eau) nécessaires à la validation des séries d'échantillons de patients qui seront réalisées. Ces contrôles sont manipulés comme des échantillons. Une série de tests comprend 6 échantillons de patients, 1 contrôle positif et 1 contrôle négatif.

Le support de RT comprend l'ensemble des réactifs nécessaires à la réalisation d'une transcription reverse (RT) : solution tampon de RT, solution de dNTPs, des amorces de RT, une solution d'inhibiteur de RNAse, une enzyme de RT et une solution contenant le contrôle interne.

Ledit support de PCR comprend un support physique de 4 aliquotes de barrettes de tubes comprenant respectivement les amorces de PCR spécifiques des miARNs hsa-miR-30a-5p, hsa-miR-376c et hsa-miR-155, auxquels s'ajoute la/les amorces par normalisateur, choisis parmi hsa-miR-126-3p, hsa-miR-146a-5p et hsa-miR-191-5p.

En complément du support physique, le support de PCR comprend les réactifs nécessaires à la PCR : Master mix de PCR.

La mise en contact des miARNs circulants de l'échantillon test avec le support comprend les étapes suivantes :
- Préparation de la solution de RT en mélangeant le support de RT
- Ajout à ladite solution de RT du contrôle interne.
- Ajout de l'échantillon de miARNs circulants dans la préparation et exécution du programme de RT tel que définis sur les appareils adéquats.
- Obtention d'un produit de RT.
- Préparation des solutions de PCR en ajoutant dans chacun des tubes du support physique de la PCR le master mix de PCR.
- Répartition de la solution de PCR obtenue dans les puits d'une plaque 96 puits adéquates à l'appareil de PCR temps réel
- Ajout à la place adéquate d'une fraction du produit de RT de l'échantillon
- Exécution du programme de PCR tel que définis sur les appareils adéquats.

### Composition et kit prédictifs de la réponse au traitement (Dworak)

Le kit de la réponse au traitement par référence Dworak comprend un support de RT pour la transcription reverse et un support PCR l'amplification.

Le support contient les contrôles positifs (sérum contrôle) et négatifs (tube d'eau) nécessaires à la validation des séries d'échantillons de patients qui seront réalisées. Ces contrôles sont manipulés comme des échantillons. Une série de tests comprend 2 à 6 échantillons de patients, 1 contrôle positif et 1 contrôle négatif.

Ledit support de RT comprend l'ensemble des réactifs nécessaires à la réalisation d'une transcription reverse : solution tampon de RT, solution de dNTPs, des amorces de RT, une solution d'inhibiteur de RNAse, une enzyme de RT et une solution contenant le contrôle interne.

Ledit support de PCR comprend un support physique de 6 aliquotes de barrettes de tubes comprenant respectivement les amorces de PCR spécifiques des miARNs hsa-miR-185, hsa-miR-376c, hsa-miR-106a, hsa-miR-25, hsa-miR-30a-5p, hsa-miR-16, hsa-let-7b et hsa-miR-19, auxquels s'ajoute la/les amorces par normalisateur, choisi parmi hsa-miR-126-3p, hsa-miR-146a-5p et hsa-miR-191-5p.

En complément du support physique, le support de PCR comprend les réactifs nécessaires à la PCR : Master mix de PCR.

La mise en contact des miARNs circulants de l'échantillon test avec le support comprend les étapes suivantes :
- Préparation de la solution de RT en mélangeant le support de RT
- Ajout à ladite solution de RT du contrôle internes.
- Ajout de l'échantillon de miARNs circulants dans la préparation et exécution du programme de RT tel que définis sur les appareils adéquats.
- Obtention d'un produit de RT.
- Préparation des solutions de PCR en ajoutant dans chacun des tubes du support physique de la PCR le master mix de PCR.
- Répartition de la solution de PCR obtenue dans les puits d'une plaque 96 puits adéquates à l'appareil de PCR temps réel
- Ajout à la place adéquate d'une fraction du produit de RT de l'échantillon de miARNs circulants
- Exécution du programme de PCR tel que définis sur les appareils adéquats.

### Composition et kit prédictifs de la réponse au traitement (IRM)

Le kit de la réponse au traitement par référence IRM comprend un support de RT pour la transcription reverse et un support PCR pour l'amplification.

Le support contient les contrôles positifs (sérum contrôle) et négatifs (tube d'eau) nécessaires à la validation des séries d'échantillons de patients qui seront réalisées. Ces contrôles sont manipulés comme des échantillons. Une série de tests comprend 6 échantillons de patients, 1 contrôle positif et 1 contrôle négatif.

Le support de RT comprend l'ensemble des réactifs nécessaires à la réalisation d'une transcription reverse : solution tampon de RT, solution de dNTPs, des amorces de RT, une solution d'inhibiteur de RNAse, une enzyme de RT et une solution contenant le contrôle interne.

Le support de PCR comprend un support physique de 4 aliquotes de barrettes de tubes comprenant respectivement les amorces de PCR spécifiques d'au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b, auxquels s'ajoute la/les amorces par normalisateur, choisi parmi choisi parmi hsa-miR-126-3p, hsa-miR-146a-5p et hsa-miR-191-5p.

En complément du support physique, le support de PCR comprend les réactifs nécessaires à la PCR : Master mix de PCR.

La mise en contact des miARNs circulants de l'échantillon test avec le support comprend les étapes suivantes :
- Préparation de la solution de RT en mélangeant le support de RT
- Ajout à ladite solution de RT du contrôle interne.
- Ajout de l'échantillon de miARNs circulants dans la préparation et exécution du programme de RT tel que définis sur les appareils adéquats.
- Obtention d'un produit de RT.
- Préparation des solutions de PCR en ajoutant dans chacun des tubes du support physique de la PCR le master mix de PCR.
- Répartition de la solution de PCR obtenue dans les puits d'une plaque 96 puits adéquates à l'appareil de PCR temps réel
- Ajout à la place adéquate d'une fraction du produit de RT de l'échantillon de miARNs circulants
- Exécution du programme de PCR tel que définis sur les appareils adéquats.

### Protocole de détection de la récidive métastatique chez un sujet

La détection de la récidive métastatique chez un sujet se fait à partir d'une intervention non invasive qui consiste en un prélèvement sanguin. Ledit prélèvement est traité dans un délai inférieur à 24 heures : il peut être congelé ou le sérum peut être directement récupéré. Ce dernier peut être soit congelé, soit subir une extraction pour récupérer les miARNs circulants. L'échantillon ou lesdits miARNs sont mis en contact avec une composition d'amorces, des sondes, ou un kit, tels que précédemment décrits, permettant la détection/quantification des miARNs hsa-miR-30a-5p, hsa-miR-376c et hsa-miR-155 et de normalisateur(s) choisi(s) parmi hsa-miR-126-3p, hsa-miR-146a-5p et hsa-miR-191-5p.

Cette mise en contact permet la détermination des miARNs de la signature prédictive de la récidive et des miARNs normalisateurs. Ladite détermination donne la prédiction pour le sujet considéré.

### Protocole de détection de la réponse au traitement chez un sujet

La détection de la réponse au traitement chez un sujet se fait à partir d'une intervention non invasive qui consiste en un prélèvement sanguin. Ledit prélèvement est traité dans un délai inférieur à 24 heures : il peut être congelé ou le sérum peut être directement récupéré. Ce dernier peut être soit congelé, soit subir une extraction pour récupérer les miARNs circulants. L'échantillon ou lesdits miARNs sont mis en contact avec une composition d'amorces, des sondes, ou un kit, tels que précédemment décrits, permettant la détection/quantification des hsa-miR-185, hsa-miR-376c, hsa-miR-106a, hsa-miR-25, hsa-miR-30a-5p, hsa-miR-16, hsa-let-7b et hsa-miR-19 et de normalisateur(s) choisi(s) parmi hsa-miR-126-3p, hsa-miR-146a-5p et hsa-miR-191-5p.

Dans une autre variante, l'échantillon ou lesdits miARNs sont mis en contact avec une composition d'amorces, des sondes, ou un kit, tels que précédemment décrits, permettant la détection/quantification d'au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b, et de normalisateur(s) choisi(s) parmi hsa-miR-126-3p, hsa-miR-146a-5p et hsa-miR-191-5p.

Cette mise en contact permet la détermination des miARNs de la signature prédictive de la réponse au traitement et des miARNs normalisateurs. Ladite détermination donne la prédiction pour le sujet considéré.

### Discussion

Les résultats montrent que des signatures de miARNs circulants extraits de sérum avant tout traitement et combinés ou non avec des critères cliniques peuvent être utilisées comme biomarqueurs afin de permettre (i) la discrimination des patients répondeurs des non répondeurs au traitement RCT néo-adjuvant avec une spécificité et sensibilité significatives, et (ii) de prédire une récidive métastatique des patients avec des performances statistiques significatives.

**Tableau 1 : Signature multivariée de la réponse au traitement avec la référence DWORAK**

| **Références** | **Accession number** | **Séquences** | **SEQ ID** | **Estimate** | **Std, Error** | **z value** | **Pr(>\|z\|)** |
|---|---|---|---|---|---|---|---|
| hsa-miR-185-5p | MIMAT0000455 | UGGAGAGAAAGGCAGUUCCUGA | 1 | 2,3733 | 1,1495 | 2,065 | 0,038958 |
| hsa-miR-376c-3p | MIMAT0000720 | AACAUAGAGGAAAUUCCACGU | 2 | -1,0502 | 0,5024 | -2,09 | 0,036594 |
| hsa-miR-106a-5p | MIMAT0000103 | AAAAGUGCUUACAGUGCAGGUAG | 3 | -4,6533 | 2,1459 | -2,168 | 0,030128 |
| hsa-miR-25-3p | MIMAT0000081 | CAUUGCACUUGUCUCGGUCUGA | 4 | 4,428 | 1,9421 | 2,28 | 0,022606 |
| hsa-miR-30a-5p | MIMAT0000087 | UGUAAACAUCCUCGACUGGAAG | 5 | -3,7216 | 1,3809 | -2,695 | 0,007036 |
| hsa-miR-16-5p | MIMAT0000069 | UAGCAGCACGUAAAUAUUGGCG | 6 | -4,3409 | 1,4317 | -3,032 | 0,00243 |
| hsa-let-7b-5p | MIMAT0000063 | UGAGGUAGUAGGUUGUGUGGUU | 7 | 2,5872 | 0,8167 | 3,168 | 0,001534 |
| hsa-miR-19a-3p | MIMAT0000073 | UGUGCAAAUCUAUGCAAAACUGA | 8 | 2,6556 | 0,8034 | 3,305 | 0,000948 |

**Tableau 2 : Analyses statistiques univariées de la réponse au traitement néo-adjuvant par référence IRM et filtrage des courbes d'amplifications**

| | | | | **[Répondeur/Non répondeur]** | |
|---|---|---|---|---|---|
| **miARN** | **Accession number** | **Séquences** | **SEQ ID** | **P-valeur** | **Fold change** |
| hsa-miR-17-5p | MIMAT0000070 | CAAAGUGCUUACAGUGCAGGUAG | 9 | 0,0075 | 1,6044 |
| hsa-miR-571 | MIMAT0003236 | UGAGUUGGCCAUCUGAGUGAG | 10 | 0,0081 | 0,3413 |
| mmu-miR-451a | MIMAT0001632 | AAACCGUUACCAUUACUGAGUU | 11 | 0,0106 | 2,3534 |
| hsa-miR-19b-3p | MIMAT0000074 | UGUGCAAAUCCAUGCAAAACUGA | 12 | 0,0125 | 1,8666 |
| hsa-miR-223-3p | MIMAT0000280 | UGUCAGUUUGUCAAAUACCCCA | 13 | 0,0126 | 1,5573 |
| hsa-miR-19a-3p | MIMAT0000073 | UGUGCAAAUCUAUGCAAAACUGA | 14 | 0,0315 | 1,6806 |
| hsa-miR-195-5p | MIMAT0000461 | UAGCAGCACAGAAAUAUUGGC | 15 | 0,0367 | 1,6135 |
| hsa-miR-106a-5p | MIMAT0000103 | AAAAGUGCUUACAGUGCAGGUAG | 16 | 0,0367 | 1,5632 |
| hsa-miR-516b-3p | MIMAT0002860 | UGCUUCCUUUCAGAGGGU | 17 | 0,0453 | 0,4911 |
| hsa-miR-16-5p | MIMAT0000069 | UAGCAGCACGUAAAUAUUGGCG | 18 | 0,0509 | 1,4534 |
| hsa-miR-1227-3p | MIMAT0005580 | CGUGCCACCCUUUUCCCCAG | 19 | 0,0761 | 0,5388 |
| hsa-miR-1290 | MIMAT0005880 | UGGAUUUUUGGAUCAGGGA | 20 | 0,0770 | 0,4815 |

| | | | | | |
|---|---|---|---|---|---|
| - expression du mir + faible dans le groupe + expression du mir + forte dans le groupe *= Méthode statistique utilisée= Mann whittney Normalisation par hsa-miR-126 et hsa-miR-191 | | | | | |

**Tableau 3 : Analyses statistiques multivariées ou signatures de la récidive métastatique**

| **Références** | **Accession number** | **Séquences** | **SEQ ID** | **Technologie : Plaque à façon** |
|---|---|---|---|---|
| hsa-miR-30a-5p | MIMAT0000087 | UGUAAACAUCCUCGACUGGAAG | 21 | 0.012244 |
| hsa-miR-376c-3p | MIMAT0000720 | AACAUAGAGGAAAUUCCACGU | 22 | 0.014390 |
| hsa-miR-155-5p | MIMAT0000646 | UUAAUGCUAAUCGUGAUAGGGGU | 23 | 0.000892 |

**Tableau 4 : miARNs de références/normalisateurs spécifiques au cancer du rectum localement avancé**

| **Références** | **Accession number** | **Séquences** | **SEQ ID** | **M-value** |
|---|---|---|---|---|
| hsa-miR-146a-5p | MIMAT0000449 | UGAGAACUGAAUUCCAUGGGUU | 24 | 0,611 |
| hsa-miR-191-5p | MIMAT0000440 | CAACGGAAUCCCAAAAGCAGCUG | 25 | 0,611 |
| hsa-miR-126-3p | MIMAT0000445 | UCGUACCGUGAGUAAUAAUGCG | 26 | 0,69 |

**Tableau 5: Observation contre Prédiction à partir de la signature pour la réponse au traitement**

| | | Observé | | |
|---|---|---|---|---|
| | | NR | R | |
| Prédit | NR | 24 | 6 | |
| | R | 6 | | 21 |
| | | | | |
| Sensibilité | 0,8 | | | |
| Spécificité | 0,778 | | | |
| IC AUC | 0,786 | 0,879 | | 0,972 |

**Tableau 6 : Description de la cohorte**

| | | **N=69** | **%** |
|---|---|---|---|
| Sexe | | | |
| | Féminin | 20 | 29.0 |
| | Masculin | 49 | 71.0 |
| Age | | | |
| | Médiane, min-max | 62 | 39-86 |
| Métastatique d'emblée | | | |
| | Non | 64 | 92.8 |
| | Oui | 5 | 7.2 |
| Dworak | | | |
| | 0 | 2 | 2.9 |
| | 1 | 15 | 21.7 |
| | 2 | 18 | 26.1 |
| | 3 | 22 | 31.9 |
| | 4 | 12 | 17.4 |
| IRM | | | |
| | 0 | 23 | 32.9 |
| | 1 | 16 | 22.9 |
| | 2 | 8 | 11.4 |
| | *Manquant* | 23 | 32.9 |
| Récidive locale | | | |
| | Non | 64 | 94.1 |
| | Oui | 4 | 5.9 |
| Récidive métastatique | | | |
| | Non | 42 | 65.6 |
| | Oui | 22 | 34.4 |

## Revendications

1. Méthode pour déterminer l'évolution clinique d'un patient atteint de cancer du rectum, en particulier un cancer du rectum localement avancé, comprenant la détermination in vitro de la présence ou de la quantité d'un ensemble de miARNs circulants dans un échantillon de fluide biologique dudit patient, ladite présence ou quantité étant indicative de l'évolution clinique du patient, ledit ensemble comprenant les miARNs suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b.

2. Méthode selon la revendication 1, pour déterminer si un patient atteint de cancer du rectum de stade II ou III est susceptible de répondre à un traitement de radiochimiothérapie combinée, comprenant la détermination in vitro, dans un échantillon biologique dudit patient, de la présence, absence, ou quantité des miARNs suivants : hsa-miR-185, hsa-miR-376c, hsa-miR-106a, hsa-miR-25, hsa-miR-30a-5p, hsa-miR-16, hsa-let-7b et hsa-miR-19.

3. Méthode selon la revendication 1, pour déterminer si un patient atteint de cancer du rectum de stade II ou III est susceptible de répondre à un traitement de radiochimiothérapie combinée, comprenant la détermination in vitro, dans un échantillon biologique dudit patient, de la présence, absence, ou quantité d'au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b.

4. Méthode selon la revendication 3, comprenant la détermination de la présence ou quantité d'au moins hsa-miR-17-5p et d'un miARN choisi parmi hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p et hsa-mir-1274b.

5. Méthode selon la revendication 3, comprenant la détermination de la présence ou quantité d'au moins hsa-miR-16-5p et d'un ARN choisi parmi hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-1227-3p, hsa-miR-1290, hsa-miR-20a-5p, et hsa-mir-1274b.

6. Méthode selon la revendication 3, comprenant la détermination de la présence ou quantité des miARNs suivants :
. hsa-miR-17-5p et hsa-miR-571,
. hsa-miR-17-5p et hsa-miR-571 et mmu-miR-451a,
. hsa-miR-17-5p et hsa-miR-195-5p,
. hsa-miR-17-5p et hsa-miR-16-5p,
. hsa-miR-16-5p et hsa-miR-195-5p,
. hsa-miR-16-5p et hsa-miR-1227-3p,
. hsa-miR-17-5p et hsa-miR-571 et mmu-miR-451a et hsa-miR-16-5p et hsa-miR-195-5p et hsa-miR-516b-3p, ou
.hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b.

7. Méthode selon la revendication 1, pour déterminer si un patient atteint de cancer du rectum de stade II ou III est susceptible de récidiver après traitement, comprenant la détermination in vitro, dans un échantillon biologique dudit patient, de la présence ou quantité de chacun des miARNs suivants : hsa-miR-30a-5p, hsa-miR-376c et hsa-miR-155.

8. Méthode selon l'une quelconque des revendications précédentes, comprenant en outre la détermination de la présence ou quantité d'au moins un miARN normalisateur choisi parmi hsa-miR-126-3p, hsa-miR-146a-5p et hsa-miR-191-5p.

9. Méthode selon l'une quelconque des revendications précédentes, **caractérisée en ce que** la détermination de la présence, absence, ou quantité de miARN est réalisée par amplification sélective au moyen d'amorces nucléiques, de préférence par PCR en temps-réel, ou par hybridation sélective au moyen de sondes.

10. Méthode selon l'une quelconque des revendications précédentes, **caractérisée en ce que** l'échantillon de fluide biologique est un échantillon de sang, plasma ou sérum, éventuellement prétraité.

11. Composition ou dispositif comprenant un ensemble de sondes nucléiques, l'ensemble de sondes comprenant au moins une sonde nucléique complémentaire et spécifique de chaque miARN des ensembles de miARNs suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux miARNs choisis parmi hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b.

12. Composition ou dispositif selon la revendication 11, **caractérisé en ce que** les sondes sont immobilisées sur une surface d'un matériau support, de préférence selon un arrangement ordonné.

13. Composition comprenant une pluralité d'amorces nucléiques, ladite pluralité d'amorces comprenant une amorce nucléique permettant l'amplification sélective d'une région au moins de chaque miARN des ensembles suivants :
. hsa-miR-185, hsa-miR-376c, hsa-miR-106a, hsa-miR-25, hsa-miR-30a-5p, hsa-miR-16, hsa-let-7b et hsa-miR-19 ; ou
. hsa-miR-30a-5p, hsa-miR-376c et hsa-miR-155 ; ou
. au moins deux microARNs choisis hsa-miR-17-5p, hsa-miR-571, mmu-miR-451a, hsa-miR-19b-3p, hsa-miR-223-3p, hsa-miR-19a-3p, hsa-miR-195-5p, hsa-miR-106a-5p, hsa-miR-516b-3p, hsa-miR-16-5p, hsa-miR-1227-3p, hsa-miR-1290, hsa-miR-20a-5p, et hsa-mir-1274b.

14. Kit comprenant un compartiment ou conteneur comprenant au moins un ensemble de sondes ou amorces tels que défini dans l'une des revendications 11 à 13 et, optionnellement, des réactifs pour une réaction d'hybridation ou d'amplification ainsi que, le cas échéant, des contrôles et/ou instructions.

15. Utilisation d'une composition ou d'un dispositif ou kit selon l'une des revendications 11 à 14 pour la détermination de l'évolution clinique d'un patient ayant un cancer du rectum.
